# EUROPÄISCHE PATENTSCHRIFT

(11) **EP 2 753 368 B1**
(45) Veröffentlichungstag und Bekanntmachung des Hinweises auf die Patenterteilung: **29.05.2019**
(21) Anmeldenummer: 12756659.4
(22) Anmeldetag: 04.09.2012
(51) Int. Cl.: A61L 15/22, A61L 15/42

(54) **VERWENDUNG EINER SEMI-OKKLUSIVEN FLEXIBLEN FLÄCHIGEN WUNDAUFLAGE ZUR BEHANDLUNG VON WUNDEN BEI TIEREN**
USE OF A SEMI-OCLUSIVE PLANAR FLEXIBLE BANDAGE FOR TREATMENT OF ANIMAL'S WOUNDS
UTILISATION D'UN PANSEMENT PLAT SEMI-OCCLUSIF POUR LE TRAITEMENT DES PLAIES DES ANIMAUX

(30) Priorität: 06.09.2011 DE 102011112433
(43) Veröffentlichungstag der Anmeldung: 16.07.2014
(73) Patentinhaber: Dr. Kenndoff GmbH & Co. KG, 21079 Hamburg (DE)
(72) Erfinder: KENNDOFF, Jochen, 21075 Hamburg (DE)
(74) Vertreter: Hausfeld, Norbert
(86) Internationale Anmeldenummer: PCT/EP2012/003690
(87) Internationale Veröffentlichungsnummer: WO 2013/034277

(56) Entgegenhaltungen:
- EP-A2- 1 190 722
- EP-A2- 1 190 723
- CA-A1- 2 539 148
- US-A1- 2010 106 107

## Beschreibung

Die Erfindung bezieht sich auf eine spezielle flächige Wundauflage zur Verwendung in der Behandlung von insbesondere kontaminierten Wunden bei Tieren. Die Wundauflage weist eine Wundkontaktschicht auf, die insbesondere aus Polyurethan, Silikon oder einer Hydrocolloidmatrix besteht und in die hydrophile Polymere eingebettet sind.

Der Anmeldungsgegenstand wird durch die Ansprüche definiert. Jeder Hinweis in der Beschreibung auf eine Behandlungsmethode ist als Wundauflage zur Verwendung in einer Behandlungsmethode zu interpretieren.

Die Verwendung von semi-okklusiven Verbänden zur Behandlung von Wunden bei Tieren, insbesondere Pferden, ist bekannt (siehe Stashak et. al. in Clinical Techniques in Equine Practice). Das beschriebene und bekannte Prinzip solcher Verbände ist, eine feuchte Wundatmosphäre zu schaffen, die die Reparaturphase der Wundheilung begünstigt. Die Verbände sind so ausgelegt, dass überschüssige Flüssigkeit durch sie hindurch nach außen verdunsten kann, aber gleichzeitig eine Dehydrierung der Wunde vermieden wird.

In derselben Publikation ist beschrieben, dass starke kontaminierte oder infizierte Wunden mit einem klebenden Verband abgedeckt werden sollten, bis sich ausreichend Granulationsgewebe entwickelt hat. Für die sich dann anschließende Reparaturphase wird die Verwendung eines semi-okklusiven Verbandes vorgeschlagen.

Einen Sonderfall stellen die z.B. im Rahmen der Mortellaro'schen Krankheit bei Kühen auftretenden Wunden dar. Diese Wunden befinden sich im Klauenbereich und sind daher in der Regel hochgradig verschmutzt und meistens mit Entzündungsprodukten belegt. Die Erkrankungsdauer ist selbst bei günstigem Verlauf relativ lang, kann zu Lahmheiten führen und in hohen Verlusten von bis zu EUR 100,00 pro erkranktem Tier resultieren.

Die bekannten und zugelassenen Behandlungsmethoden basieren auf echten Arzneimitteln z.B. antibiotikahaltigen Arzneimittel, die aufgesprüht werden (CTC-Spray oder auch Blauspray genannt) mit Chlortetracyclin als Wirkstoff. Hier besteht für den Anwender, der häufig oder berufsmäßig damit arbeiten muss, die Gefahr, den antibiotikahaltigen Sprühnebel einzuatmen und so Resistenzen auf die Wirkstoffklasse zu entwickeln. Außerdem sind die einzuhaltenden Bedingungen nach der Anwendung (das Tier sollte mindestens 1 Stunde auf trockenem Untergrund stehen) unrealistisch, da die Tiere in aller Regel nur in ihre normale, im Bereich der Klauen feuchte, Stallumgebung entlassen werden können.

Eine andere Behandlungsmethode beruht auf salicylhaltigen Formulierungen (z.B. Novaderma), die in der Regel mit einem Verband aufgebracht werden. Hier sind zumeist mehrfache aufeinanderfolgende Anwendungen erforderlich, da andernfalls keine ausreichende keratolytische und antibakterielle Wirksamkeit gegeben sind. Ausserdem ist diese Behandlungsmethode mit einer Wartezeit für die Abgabe der Milch von einem Tag nach Abnahme des Verbandes belegt.

Gängig sind auch Klauenbäder unter Einsatz von aggressiven Lösungen, wie z.B. Formalinlösung, Kupfersulfatlösung oder chlorhaltigen Lösungen z.B. schwach konzentrierte Perchlorsäurelösung, um nur einige Beispiele zu nennen. Diese sind oft erst in Konzentrationen wirksam, die für die Anwendung nicht mehr zulässig sind.

Alternativ oder ergänzend sind Antibiotika-Fußbäder bekannt.

Auch die parenterale Gabe von Antibiotika z.B. Cefquinom ist bekannt und als wirksam beschrieben. Allerdings ist auch hier nach dem Absetzen eine Wartezeit von 1 Tag bei Milch und 7 Tage beim Fleisch einzuhalten.

Aus der DE 10 2009 057 267 ist die Verwendung einer speziellen Salbe in Verbindung mit einer Vorbehandlung mit Blauspray bekannt. In der Anwendungsbescheibung des dazugehörigen Verkaufsproduktes (Mortekill) wird ausgeführt, dass der Wundbereich angezündet wird, bevor die Salbe aufgebracht wird.

Weiterhin werden auch Vereisungssprays für die Behandlung diskutiert und in der Praxis versucht.

Abflammen und Vereisen führen jedoch zu oberflächlichen Brandwunden bzw. brandwundenähnlichen Erfrierungswunden, die in aller Regel später in deutlich größeren Läsionen resultieren.

Problematisch an den meisten genannten Methoden ist auch, dass das Aufbringen von Lösungen, beziehungsweise Salben, nur über eine begrenzte Zeit wirksam ist (nämlich solange, wie die Salbe, beziehungsweise die Lösung, auf die Wunde einwirken kann, bevor sie durch den Kontakt der Klaue mit der Umwelt abgewaschen, beziehungsweise abgelöst wird). Daher wird oftmals zusätzlich ein Verband angelegt, um die Mittel länger auf der kranken Haut zu halten. Die üblicherweise verwendeten kohäsiven oder nicht kohäsiven Gewebebinden sind jedoch durchlässig für die Exkremente aus dem Stall und saugen sich mit der Zeit mit diesen voll, sodass der behandelte Bereich trotzdem weiterhin einer Verschmutzung von außen ausgesetzt ist. Das führt dazu, dass dann eine Behandlung ggf. 2 oder 3 mal wiederholt werden muss.

Aus der DE 29611414U1 sind schließlich auch nicht durchlässige Verbände zur Behandlung von Klauenkrankheiten bei Rindern bekannt, die aus einer insbesondere Bitumen enthaltenden Paste bestehen. Anzunehmen ist aber, dass mit einem solchen Verband okklusive Bedingungen eingestellt werden, die eine Wundheilung nur begrenzt befördern. Okklusive Bedingungen sind grundsätzlich weniger geeignet, da es sich bei den in diesen Wunden nachgewiesenen Keimen in der Regel um anaerobe Bakterienstämme handelt.

In der Patentschrift US2010/010610 wird eine Wundauflage zur Behandlung von kontaminierten Wunden bei Tieren, welche eine semi-okklusive Membran mit einem aktiven Wirkstoff enthält, beschrieben.

Aufgabe der Erfindung ist es, eine Behandlungsmöglichkeit zu bieten, die die zuvor beschriebenen Nachteile der derzeit existierenden Behandlungsformen umgeht.

Gelöst wird diese Aufgabe gemäß Anspruch 1.

Erfindungsgemäß ist vorgesehen, die genannten problematischen Wunden, wie sie insbesondere als Mortellaro'sche Krankheit bei Kühen oder z.B. Mauke bei Pferden bekannt sind, mit einer speziellen semi-okklusiven Wundauflage zu behandeln, die eine Wundkontaktschicht aus einer elastomeren oder thermoplastisch elastomeren Komponente aufweist, in die hydrophile Polymere eingebettet sind.

Eine besonders geeignete Wundauflage ist weiter unten, in Beispiel 1, beschrieben.

Es wurde überraschend festgestellt, dass sich Wunden in unterschiedlichen Stadien (akut aktiv, chronisch aktiv, in Abheilung befindlich, in Ruhe befindlich, etc.) gleichermaßen gut mit der erfindungsgemäßen Wundauflage behandeln lassen. Die erfindungsgemäße Behandlung der Wunde durch Abdeckung mit einer speziellen Wundauflage fördert sowohl die Bildung von Granulationsgewebe bei tieferen Defekten als auch die sich anschließende Epitelisierungsphase, was im Hinblick auf die insbesondere in Verbindung mit der Behandlung der Mortellaro'schen Krankheit beschriebenen Probleme keinesfalls zu erwarten war.

Besonders überraschend ist, dass ohne Zusatz von Medikamenten die erfindungsgemäße Verwendung der Wundauflage, wie weiter ausgeführt, auch unter den üblichen Haltungsbedingungen der Tiere, insbesondere der Rinder, möglich ist (Nässe, Schmutz) und zu raschen unproblematischen Heilungserfolgen führt. In der Regel reicht es, die Wunde mit der erfindungsgemäß verwendeten Wundauflage flächig abzudecken und diese dann mit einem weiteren Verband an der Klaue so zu sichern, dass der Kontakt der Wundauflage zur Wundoberfläche bzw. zum Wundsekret gewährleistet ist und durch eine Fixierung über mehrere Tage in dieser Position gehalten wird. Gleichzeitig wird durch die Fixierung die Wundauflage auf die noch intakte Haut aufgepresst, sodass nachträglich kein Schmutz mehr unter die Wundauflage fließen kann. Die semi-okklusive Eigenschaft des Materials verhindert, das Schmutz und Bakterien durch die Wundauflage hindurch in den Wundbereich vordringen können, der abgedeckte Bereich aber nach wie vor atmen kann. Der so durch den körpereigenen Reinigungsprozess gesäuberte Wundbereich kann so nicht erneut von außen kontaminiert werden und der tierische Organismus kann ungestört eine intakte Hautbarriere aufbauen. Auch wird so gewährleistet, dass kein anaerobes Millieu entstehen, in denen etliche der schädlichen Keime besonders gut gedeihen könnten. Auch die gesamte Problematik, die sich durch Antibiotikaeinsatz ergibt wie sich bildende Antibiotikaresistenzen oder Gefährdungen der Anwender wird hierdurch elegant umgangen.

Mit der Erfindung lassen sich die Nachteile bekannter Behandlungen in einfacher Weise beheben.

Durch die Verwendung einer die Wunde kontaktierenden semi-okklusiven Wundauflage lassen sich die üblicherweise stark kontaminierten und gegebenenfalls entzündlichen Wunden effektiv gegen weitere Verschmutzungen von außen schützen. Die Wundauflage bildet eine Art Schorfersatz, der die Wunde vor weiterer Kontamination von außen schützt. Ein ggf. (bei Mauke) gebildeter harter Schorf wird aufweicht bzw. ein Aufreißen eines ggf. gebildeten harten Schorfs wird verhindert.

Da unter der Auflage keine anaeroben Bedingungen vorliegen, wird da ein Überleben der besonders kritischen anaeroben Bakterienstämmte erschwert bzw. verhindert. Dem tierischen Organismus wird die Möglichkeit gegeben, ein heilungsförderndes Millieu auf der Wunde zu schaffen und zu erhalten. Nach einer mechanischen Vorreinigung durch den Anwender kann die Selbstreinigung des Wundbereichs durch den Organismus des Tieres von noch oder bereits in der Wunde befindlichen Keimen und Verschmutzungen einsetzen und die Ausbildung einer intakten Hautbarriere erfolgen.

Überraschend hat sich gezeigt, dass eine einmalige Behandlung ausreichend ist.

Ein ganz wesentlicher Vorteil der Erfindung ist, dass zur Behandlung der z.B. Mortellaroschen Krankheit keine, insbesondere verschreibungspflichtigen Arzneimittel erforderlich sind. Insbesondere der Verzicht auf die üblicherweise eingesetzten Antibiotika verhindert die Bildung von Resistenzen beim Tier und Anwender. Ein weiterer wesentlicher Vorteil ist, dass die Behandlung wartezeitfrei ist, mit anderen Worten der Tierhalter kann das Tier ohne Unterbrechung nutzen, und es ist auch nicht erforderlich, dass das Tier nach und während der Behandlung unter unrealistischen Bedingungen gehalten werden muss.
Von hoher praktischer Bedeutung ist, dass der Verband als nicht zulasungspflichtiges Arzneimittel nicht durch den Tierarzt verschrieben werden muss und damit von einschlägigen Berufsgruppen wie z.B. den Klauenpflegern oder dem Landwirt selbst problemlos direkt bezogen und angewendet werden kann.

Wie oben ausgeführt, besteht die Wundkontaktschicht aus einer feuchtigkeitsdurchlässigen Matrix insbesondere z.B. aus hydrophilem Polyurethan. Darin eingebettet sind hydrophile Polymere, die Flüssigkeit binden können, wie z.B. Pektin, Gelatine, Carboxymethylcellulose oder Superabsorber.

Die Wundkontaktschicht der Wundauflage kann sowohl geschäumt als auch nicht geschäumt, klebend oder nicht klebend, sein.

Es ist möglich, die Auflage mit Zug oder auch zugfrei aufzubringen. Wichtig ist lediglich, dass der Kontakt der Wundauflage zur Wundoberfläche bzw. dem Wundsekret gewährleistet ist, so dass sich das gewünschte feuchte Wundheilungsklima einstellen kann und eine Kontamination von außen unter den Verband gelangen kann.

Weiterhin wichtig ist, dass das Material so ausgeführt ist, dass kein Schmutz und keine Bakterien durch den Verband hindurch in den Wundbereich gelangen können.

Bevorzugt ist die erfindungsgemässe verwendete Wundauflage ein Verbund aus einer Wundkontaktschicht mit einem einseitig darauf angeordneten flächigen Träger. Die Wundkontaktschicht weist eine plastische und/oder elastische Verformbarkeit auf, die gewährleistet, dass die Wundkontaktschicht sich beim Anlegen von dem Wundauflage an die gegebene Kontur anpassen kann. Idealerweise wird eine Dicke gewählt, die den Wundbereich zusätzlich ausreichend polstern kann. Auch kann der Träger der Wundkontaktschicht polsternde Eigenschaften aufweisen (z.B. ein druckentlastender Schaum) und die Wundkontaktschicht selbst nur sehr dünn ausgeführt sein. Zwingend notwendig ist die Polsterung allerdings nicht. Auf jeden Fall sollte die Oberfläche des Trägers so beschaffen sein, dass das Material die Reibung zwischen Wundbereich und Klauenhorn reduzieren kann, sodass auch bei Anwendung insbesondere im Zwischenklauenspalt eine zusätzliche Reizung vermieden wird.

Bevorzugt werden bei der erfindungsgemäss verwendeten Wundauflage Dicke und Verformbarkeit der Wundkontaktschicht und/ oder des Trägers so gewählt, dass trotz lokaler Dehnung des Verbandes eine ausreichend dicke Schicht den Wundbereich polstert.

Vorzugsweise beträgt die Dicke der eingesetzten Wundkontaktschicht und/oder des Trägers 50 - 3000 µm, bevorzugt 150 - 2000 µm, besonders bevorzugt 250 - 1000 µm. Die Verformbarkeit der Wundkontaktschicht sollte bevorzugt so beschaffen sein, dass sich der Träger eines auf Stahl flächig verklebten Filmes um mindestens 100% seiner Höhe bevorzugt mehr 300 % besonders bevorzugt mehr als 500 % vom Rand zur Mitte des Pflaster hin schieben lässt. Dies ist am Deutlichsten bei einem selbstklebenden Produkt, das auf einer Stahlplatte verklebt ist und sich bei der angegebenen Verschiebung nicht von der Stahlplatte löst. Mit anderen Worten der Träger lässt sich bei einer 1 mm dicken Klebeschicht in dieser Ausgestaltung um mindestens 1 mm, bevorzugt um > 3 mm, besonders bevorzugt um > 5 mm zurückschieben.

Weiterhin bevorzugt ist vorgesehen, dass die Wundkontaktschicht mit einer Elastizität ausgestattet ist, die besonders bevorzugt so eingestellt ist, dass sich nach Freigabe des Trägers bei der oben angegebenen Untersuchungsmethode, die Klebeschicht wieder in ihre ursprüngliche Form rückverformt.

Bei zu geringer Verformbarkeit bzw. Elastizität, kann es vorkommen, dass die Wundkontaktschicht trotz eines von außen angelegten fixierenden Verbandes keinen Kontakt zur Wundoberfläche hat. Die Folge ist, dass sich in diesem Fall kein heilungsförderndes feuchtes Wundmilieu auf der Wundoberfläche ausbilden kann und Verunreinigungen von außen unter den Verband fließen können.

Darüber hinaus sollte das Material die Reibung zwischen Wundbereich und Klauenhorn reduzieren, sodass auch bei Anwendung insbesondere im Zwischenklauenspalt eine zusätzliche Reizung vermieden wird. Idealerweise ist die Wundkontaktschicht oder der Trägerfilm so ausgestaltet, dass zusätzlich eine Polsterung des Wundbereichs gegeben ist.

Dicke, Elastizität und ggf. plastische Verformbarkeit der Wundkontaktschicht sind daher vorteilhaft so aufeinander abgestimmt, dass beim Stehen und Laufen des Tieres einwirkende Reibungskräfte kompensiert werden können.

Besonders vorteilhaft ist weiterhin, wenn nicht nur die Wundkontaktschicht sondern die Wundauflage insgesamt so ausgebildet ist, dass sie diesen Anforderungen genügt.

Im Folgenden soll insbesondere auf weitere Details der Wundkontaktschicht eingegangen werden.

Im Rahmen der Erfindung bevorzugte Wundkontaktschichten können nicht klebend sein oder weisen Klebkräfte auf Stahl > 0,5 N/cm, bevorzugt, > 0,7 N/cm, besonders bevorzugt >1 N/cm auf. Die angegebenen Klebkräfte beziehen sich auf Klebkraftmessungen auf standardisierten Stahlplatten. Es handelt sich dabei um ein dem Fachmann bekanntes Standardverfahren.

Weiterhin sollte die erfindungsgemäss eingesetzte Wundkontaktschicht ausreichend wasserdampfdurchlässig sein, damit die darunter liegende Haut insbesondere nach längerer Tragedauer nicht mazeriert. Mazeration ist nicht erwünscht, da hierdurch die die Haut gegenüber dem Angriff von Keimen deutlich anfälliger wird.

Geeignet sind Wundkontaktschichten, die per se wasserdampfdurchlässig sind, Poren enthalten können oder in die feuchtigkeitsabsorbierende Füllstoffe eingearbeitet sind.

Als Wundkontaktschichten werden daher insbesondere Polyurethanklebeschichten, Silikonklebeschichten oder Hydrokolloidklebeschichten bevorzugt, die zusammen mit dem Träger eine Wasserdampfdurchlässigkeit von mind. 300 g/m² in 24 h, bevorzugt 500 g/m² in 24 h oder mehr aufweisen und/oder mindestens eben diese Mengen an Feuchtigkeit von der Hautoberfläche innerhalb dieses Zeitraumes aufnehmen und in der Wundkontaktschicht und/oder dem Träger zu binden vermögen.

Erfindungsgemäß ist vorgesehen, dass die Wundkontaktschicht in Kontakt mit einer feuchten Wundoberfläche nicht klebt oder verklebt. Vielmehr erhält sie stattdessen ein Feuchtigkeitsmilieu auf der Wundoberfläche vergleichbar dem unter einem natürlichen Schorf. Die Wundkontaktschicht verklebt so nicht im Wundbett und fördert damit eine beschleunigte Abheilung der Wunde.

Die Wundkontaktschicht sollte darüber hinaus ein möglichst geringes Sensibilisierungspotential aufweisen.

Von wesentlicher Bedeutung ist die Sauerstoffdurchlässigkeit der Wundauflage, damit auch die mehrere Tage abgedeckte Haut durch den Verband hindurch weiterhin atmen kann und anaerobe Bakterien nicht wachsen können. Andernfalls könnte die Haut gereizt werden oder anaerobe Bakterien sich ungehindert vermehren. Dies trägt mit dazu bei, dass man die Wundauflage längere Tage auf der Wunde belassen kann, was wiederum den Schutz des Wundbereichs verbessert und das heilungsfördernde Milieu unterstützt.

Von Bedeutung ist auch, dass die erfindungsgemäß verwendete Wundkontaktschicht Wundsekret unter Druck aufnehmen kann und nicht wieder abgibt. Dies kann man erreichen, indem man der Klebstoffschicht Superabsorber-Partikel oder andere geeignete hydrophile Polymere beimischt, wie sie z.B. aus der Windelherstellung bekannt sind. Geeignete Partikel sind z.B. unter dem Namen FAVOR T 5233 im Handel erhältlich.

Optimiert werden können die erfindungsgemäss verwendeten Wundauflagen durch vorteilhafte Ausgestaltungen des Trägers.

Grundsätzlich geeignete Träger können alle aus allen flexiblen flächigen Materialien bestehen, die einen flächigen Zuschnitt erlauben und z.B. die aus dem Gebiet der Pflaster- oder Verbandtechnik bekannten Eigenschaften aufweisen. Hier zum Einsatz kommende Träger sind beispielsweise Gewebe, Gewirke, Vliese und Folien sowie Kombinationen aus den Materialien. Geeignete Folien sind z.B. Polyethylenfolien, Polyurethanfolien, Copolyesterfolien, Polyamidfolien und Coextrudierte Folien. Geeignete Vliese sind z.B. Celluloseacetate, Polyestervliese oder Polyamidvliese. Es handelt sich bei den vorstehenden Angaben um eine nicht abschließende Aufzählung einiger Beispiele. Selbstverständlich sind noch eine Reihe weiterer dem Fachmann bekannte Materialien zur Herstellung als Träger geeignet.

Gemäss einer vorteilhaften Ausgestaltung wird ein Träger eingesetzt, der flächig dehnbar ausgebildet ist. Die Dehnung sollte so gewählt sein, dass der Träger mit der Wundkontaktschicht auf mindestens auf > 50 % bevorzugt > 100% seiner Erstreckung in dieser Richtung dehnen lässt. Besonders bevorzugt ist, wenn sich Träger und Wundkontaktschicht im Wesentlichen synchron dehnen lassen.

Die Dehnung des Trägers kann sowohl durch Recken als auch elastisches Dehnen mit allen dazwischen liegenden prozentualen Verhältnissen zwischen Reckung und Elastizität erfolgen.

Bevorzugt ist vorgesehen, dass der Träger längs und quer zur Anwendungsfläche dehnbar ist. Auch hier kann die Dehnung wieder auf Elastizität oder Reckung zurückzuführen sein.

Die der Wundkontaktschicht abgewandte Oberfläche des Trägers sollte so beschaffen sein, dass sie die Reibung zwischen Wundoberfläche und Horn insbesondere im Zwischenklauenspalt reduziert. Als Träger bevorzugt sind daher makroskopisch glatte Folien wie z.B. Polyurethan-, Polyethylen oder Polyesterfolien oder Verbünde aus mehreren unterschiedlichen Materialien, die sichtbar glatt, keimdicht und flexibel sind. Weniger geeignet, aber gleichfalls möglich sind aus diesem Grunde stärker strukturierte Oberflächen wie z.B. Gewirke, Gewebe oder Vliese, auch wenn sie hydrophobiert sind.

Der Träger ist notwendigerweise selbst oder in Verbindung mit der Wundkontaktschicht keimdicht und bleibt dies auch bei Dehnung bzw. Reckung.

In der Regel ist es erforderlich, dass die Wundauflage mit einem Zusatzverband fixiert und an den Wundbereich ggf. angedrückt wird.

Idealerweise erfolgt die Fixierung durch elastische, kohäsive Binden, elastische "Schuhe", die übergezogen werden können oder auch für Abdeckung und Fixierung im Bereich der Klaue gestaltete Produkte wie z.B. Cowslipper. Auch nicht elastische Binden können genutzt werden.

Eine besonders bevorzugte Wundauflage weist einen Träger auf, beschichtet mit einer selbstklebenden Polymermatrix als Wundkontaktschicht. Als Beispiel einer für die Anwendung relevanten Ausführungsform einer Klebeschicht gelten Qualitäten, wie sie in Patent EP 0 897 406 hinreichend beschrieben sind. Der für die erfindungsgemäße Anwendung bevorzugte Vernetzungsgrad, charakterisiert als Isocyanatkennzahl wie beschrieben in Patent EP 0 897 406, liegt bevorzug in Bereich von 41- 47, ohne jedoch die im vorgenannten Patent genannten Kennzahlbereiche ausschließen zu wollen. Der in diesem Patent beschriebenen Polymermatrix werden bevorzugt 5 - 20 % eines Füllstoffes bezogen auf die eingesetzte Menge an Polyol zugesetzt, der in der Lage ist wässrige Flüssigkeiten zu absorbieren und zu binden, ohne jedoch die im vorgenannten Patent sowie in Patent EP 0 665 856 genannten Füllstoffmengen und -Qualitäten ausschließen zu wollen. Die so charakterisierte Klebeschicht ist mit einem hochflexiblen, wasserdampf- und sauerstoffdurchlässigem Polyurethanfilm abgedeckt, ohne jedoch die in den vorgenannten Patenten genannten alternativen Wundauflage ausschließen zu wollen. Abgedeckt wird das Produkt mit handelsüblichem, dem Fachmann für selbstklebende oder nicht klebenden Produkten geläufigen Trenn- oder Schutzpapieren oder Trenn- oder Schutzfolien, die vor der Anwendung entfernt werden müssen, um die Wundkontaktseite bis zum Zeitpunkt der Anwendung zu schützen.

Das Produkt für die erfindungsgemäße Anwendung kann aus flächigem Material heraus gestanzt oder geschnitten als Einzelpflaster oder von einer Rolle abgeschnitten angewendet werden. Letzteres ermöglicht durch Abschneiden eines der Wundengröße angemessenen Pflasterstücks. Pflaster können aber auch einzeln gegossen werden. Beispielhaft seien Verfahren genannt, wie sie in der Patentanmeldung EP 1 695 721 beschrieben sind.

Überraschend für den Fachmann ist, dass der im Rahmen der Erfindung beschriebenen Wundauflage unter den extremen hygienischen Bedingungen oberflächliche Wunden ohne medikamentöse Behandlung zur Abheilung bringen kann.

Angewendet werden kann das erfindungsgemässe Verfahren bzw. der Film bei allen Tieren, die an derartigen oberflächlichen Wunden leiden, wie z.B. Kühe, Pferde etc., die sowohl in akuter, ruhender oder chronischer Form von z.B. Mortellaro- oder Mauke-ähnlichen Läsionen leiden.

Im Folgenden soll die Erfindung anhand eines Beispiels für eine geeignete Wundauflage und einer Figur, die grob schematisch eine Klaue mit angelegter Wundauflage zeigt, sowie von Versuchsergebnissen näher erläutert werden.

### BEISPIEL FÜR EINE GEEIGNETE WUNDAUFLAGE:

Eine geeignete Wundauflage ist aufgebaut aus einer etwa 40 µm starken hochflexiblen, wasserdampf- und sauerstoffdurchlässigen sowie keimdichten Polyurethanfolie (Applica, Smith & Nephew) als Träger, der beschichtet ist mit einer selbstklebenden 300 µm dicken hochflexiblen Polyurethanklebeschicht als Wundkontaktschicht.

Die Klebeschicht wurde hergestellt, indem man 100 Gew.-Anteile an Polyol (Levagel VP KA 8732; OH-Zahl 35) mit 12 Gew.-Teilen an Superabsorber (Favor T 5233), 0,1 Gew.-Teilen Katalysator (Coscat 83) und 0,8 Gew.-Teilen Vitamin E (Irganox E 201) in einer 1-1-Apparatur 2 h bei Raumtemperatur homogenisiert. Zu 100 Gew.-Teilen dieser Mischung wurden dann 6,6 Gew.-Teile an Vernetzer (Desmodur E 305, NCO-Gehalt 12,2 %) gegeben und diese intensiv mit einem Glasstab 1 Minute vermischt.

Diese Mischung wird dann auf ein handelsübliches, silikonisiertes Trennpapier gegossen, mit der als Träger dienenden Polyurethanfolie abgedeckt und mit Hilfe eines Rakels flächig verteilt, sodass der Verbund eine Dicke von etwa 300 µm aufweist und dann in einem Trockenschrank 18 Minuten bei 80°C gehärtet. Es entsteht eine transparente Wundauflage.

Aus dem entstehenden flächigen Gebilde werden Streifen von z.B. 5 cm Breite herausgeschnitten. Die Breite kann natürlich variieren. Ein Streifen mit einer Breite von 5 cm ist beispielweise gut geeignet, um die übliche Größe auftretender Läsionen im Bereich des Übergangs vom Kronsaum zum Ballen, der Fesselbeuge und des Zwischenklauenspalts abzudecken.

Die Länge des Streifens sollte mit etwa 16 cm gewählt werden, so dass mit dem Streifen der Bereich des Übergangs vom Kronsaum zum Ballen, der Fesselbeuge und des Zwischenklauenspalts mit einem Produkt abgedeckt werden kann. Befindet sich die Läsion nur im Bereich des Übergangs vom Kronsaum zum Ballen oder der Fesselbeuge, so reicht auch eine runde oder eckige Form mit 5 auf 5 cm. Das Trennpapier wird von der Wundkontaktschicht abgezogen und die Wundauflage ohne Spannung auf den Wundbereich gelegt. Ein länglicher Streifen von 5 x 16 cm wird in den Zwischenklauenspalt so eingelegt, dass sowohl Material zur Abdeckung im Bereich der Fesselbeuge oder im Bereich des Kronsaums übersteht und Kontakt zur Haut- bzw, Wundoberfläche im Zwischenklauenspalt hat. Der Überstand soll in der Lage sein Läsisonen im Bereich der Fessselbeuge oder im Bereich des Kronsaums abzudecken. Eine Läsion nur im Bereich der Fesselbeuge oder nur im Bereich des Übergangs vom Kronsaum zum Ballen kann mit einer Wundauflage der Größe 5 x 5 cm abgedeckt werden. In jedem Fall muss sichergestellt sein, dass die Wundauflage alle bei der Reinigung freigelegten Läsionen bedeckt. Gegebenenfalls können mehrere Verbände so überlappend aufgelegt werden, dass zumindest alle freigelegten Läsionen durch die Wundauflage abgedeckt werden. Die Fixierung erfolgt ggf. nach zusätzlichem Auflegen eines Polsters im Bereich der Fesselbeuge, um den Kontakt der Wundauflage mit der Wundoberfläche sicherzustellen, mit Hilfe einer kohäsiven Binde (z.B. Eurofarm kohäsive Binden)

Die Wundauflage kann nun einmal so angelegt und fixiert etwa 10 Tage auf der Klaue verbleiben. Durch den Verband gehalten bleibt sie in Position und löst sich nicht ab.

Wenn die Hautbereiche der Klaue eine Läsion aufweist, so wird die Wundauflage, genau wie oben beschrieben, angelegt. Hierbei übernimmt die Wundauflage die Funktion eines Schorfes und schützt die Wunde vor Verunreinigung und Keimen. Überschüssige Wundflüssigkeit wird langsam durch die Wundauflage absorbiert. Zum Abnehmen wird der kohäsive Verband einfach aufgeschnitten und zusammen mit der Wundauflage entfernt.

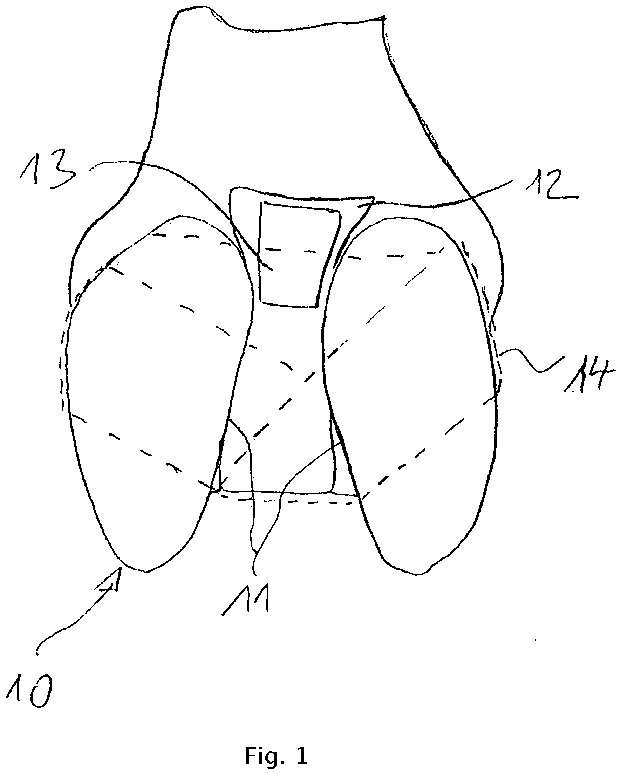

In der einzigen Figur ist grob schematisch eine Klaue 10 mit Zwischenklauenspalt 11 in Draufsicht dargestellt. In Bereich des Zwischenklauenspalts 11 befindet sich eine nicht erkennbare Wunde, die gemäss obigem Beispiel mit einer Wundauflage 12 abgedeckt ist. Um die Anlage der Wundauflage zu erhöhen, wird auf ihr ein Polster 13 angeordnet und Wundauflage 12 und Polster 13 dann mit einer elastischen Binde 14 fixiert, die mehrfach um die Klaue gewickelt wird.

### VERGLEICHSVERSUCHE:

Zum Beleg der Wirksamkeit der Erfindung wurden mehrere erkrankte Tiere mit unterschiedlichen Produkten behandelt. Die Ergebnisse nach 8 Tagen Behandlungsdauer sind in der nachfolgenden Tabelle zusammengefasst:

**Tabelle**

| **Produkt** | **Zuschnitt** | **Tier** | **Wundenposition** | **Status zu Beginn der Behandlung** | **Erfolg nach 8 Tagen Behandlung** |
|---|---|---|---|---|---|
| Beispiel | 5x16 cm | 648 | HL b | DD cranial aktiv und inaktiv, L | Limax abgeheilt |
| Beispiel | 5x16 cm | 656 | HRb | DD aktiv auf L | DD abgeheilt; L noch vorhanden |
| Beispiel | 5x16 cm | 681 | HR b | DD aktiv auf L | DD, L abgeheilt |
| Beispiel | 5x16 cm | 760 | HR b | DD aktiv | in Abheilung |
| Beispiel | 5x16 cm | 760 | HR c | DD aktiv | in Abheilung |
| Beispiel | 5x16 cm | 786 | VRc | DD aktiv | abgeheilt |
| Beispiel | 5x16 cm | 33936 | HRb | DD aktiv | in Abheilung |
| Beispiel | 5x16 cm | 82391 | HR a/b/c | DD aktiv | in Abheilung |
| Beispiel | 5x16 cm | 82392 | HL c | DD aktiv | in Abheilung |
| Beispielvariante variiert: dünner, klebend, geschäumt, erhöhter Anteil an Superabsorber | 5x6 cm | 773 | HR c | DD aktiv | in Abheilung |
| Gelbildender Hvdrocolloidverband | 5x5 cm | 802 | HR c | DD aktiv | in Abheilung |
| Beispielvariante variiert: dicker, klebend, ungeschäumt, stark erhöhter | 5x6 cm | 773 | VL Sohle | SWG aktiv | Lederhaut auf normales Niveau angehoben = Defektausfiillung |
| | | | | | |
| Hydrohob beschichtete Kompresse | 5x5 cm | 766 | HL a/dorsal | DD aktiv | Nicht in Abheilung |
| Hydrogel | 5x5 cm | 772 | HL c | DD voll aktiv | Nicht in Abheilung |
| Superabsorberhaltige Kompresse | 5x5 cm | 772 | HR c | DD aktiv | Nicht in Abheilung |
| Fettgaze | 5x5 cm | 786 | HL c | DD aktiv | aktiv |
| Alginat | 5x5 cm | 794 | HR c | DD aktiv | aktiv |
| PU-Schaum | 5x5 cm | 802 | HL c | DD aktiv | aktiv |
| Silikon-Schaumverband | 5x5 cm | 33936 | HL a | DD aktiv | Nicht in Abheilung |
| PU-Schaum | 5x5 cm | 33936 | HR c | DD aktiv | aktiv |

| | | | | | |
|---|---|---|---|---|---|
| **DD = Dermatitis Digitalis;L = Limax; SWG = Sohlenwandgeschwür; HL, HR, VL, VR = Hinten Links, Hinten Rechts, Vorne Links, Vorne Rechts; a = Übergang Kronsaum zum Ballen; b = ZKS (Zwischenklauenspalt); c** = **Fesselbeuge; d** = **Klauensohle** | | | | | |

Man erkennt, dass die Verwendung der im Beispiel beschriebenen Wundauflage (Beispiel) bei allen behandelten Tieren (648-82392) zu einer erfolgreichen Abheilung führte. Ebenfalls gute Wirkung zeigten die Beispielvarianten sowie ein gelbildendender Hydrocolloidverband.

Nicht wirksam waren dagegen die bei den Tieren 766 bis 33936 eingesetzten Verbände. Hier war nach 8 Tagen keine Abheilung zu beobachten.

## Patentansprüche

1. Semi-okklusive flexible flächige Wundauflage mit einer Wundkontaktschicht bestehend aus einer elastomeren oder thermoplastisch elastomeren Komponente, in die hydrophile Polymere eingebettet sind, zur Verwendung in der Behandlung von kontaminierten Wunden bei Tieren, insbesondere im Bereich der Gliedmassen.

2. Wundauflage zur Verwendung nach Anspruch 1, **dadurch gekennzeichnet, dass** es sich um Wunden handelt, die bei auch nur geringfügiger Verletzung der Haut durch ungünstige, zu feuchte Haltungsbedingungen, mangelnde Hygiene, genetisch bedingte ungünstige Bedingungen oder in Verbindung mit Stress z.B. die Mortellaro'sche Krankheit (Kuh) oder Mauke (Pferd) hervorgerufen werden.

3. Wundauflage zur Verwendung nach Anspruch 1, **dadurch gekennzeichnet, dass** die Wundkontaktschicht aus Polyurethan, Silikon oder einer Hydrocolloidmatrix besteht.

4. Wundauflage zur Verwendung nach Anspruch 1, **dadurch gekennzeichnet, dass** die Wundkontaktschicht selbstklebend ausgebildet ist.

5. Wundauflage zur Verwendung nach Anspruch 1, **dadurch gekennzeichnet, dass** die eingebetteten hydrophilen Polymere hydrophile flüssigkeitsbindende Partikel wie z,B. Pektin, Gelatine, Carboxymethylcellulose oder Superabsorber sind.

6. Wundauflage zur Verwendung nach Anspruch 1, **dadurch gekennzeichnet, dass** die Wundauflage einen atmungsaktiven (semi-okklusiven) Kunststofffilm als Aussenschicht aufweist.

## Claims

1. Semi-occlusive, flexible, planar wound dressing comprising a wound contact layer consisting of an elastomeric or thermoplastic elastomeric component in which hydrophilic polymers are embedded, for use in the treatment of contaminated wounds in animals, particularly in the region of the limbs.

2. Wound dressing for use according to claim 1, **characterized in that** the wounds are wounds caused, in the event of only minor injury to the skin, by adverse living conditions that are too damp, a lack of hygiene, genetically determined adverse conditions or in conjunction with stress, for example by Mortellaro's disease (cows) or mud fever (horses).

3. Wound dressing for use according to claim 1, **characterized in that** the wound contact layer consists of polyurethane, silicon or a hydrocolloid matrix.

4. Wound dressing for use according to claim 1, **characterized in that** the wound contact layer is self-adhesive.

5. Wound dressing for use according to claim 1, **characterized in that** the embedded hydrophilic polymers are hydrophilic liquid-binding particles, such as pectin, gelatin, carboxymethylcellulose or superabsorbers.

6. Wound dressing for use according to claim 1, **characterized in that** the wound dressing has a breathable (semi-occlusive) plastics film as an outer layer.

## Revendications

1. Pansement semi-occlusif flexible plat avec une couche de contact avec la plaie constituée d'un composant élastomère ou élastomère thermoplastique enrobant des polymères hydrophiles, destiné au traitement de plaies contaminées chez les animaux, en particulier au niveau des membres.

2. Pansement pour une utilisation selon la revendication 1, **caractérisé en ce qu'**il s'agit de plaies qui, en cas de blessure même légère de la peau, sont provoquées par des conditions d'élevage défavorables et trop humides, une hygiène insuffisante, des conditions génétiquement défavorables ou en relation avec un stress causé par ex. par la maladie de Mortellaro (vache) ou l'eczéma du paturon (cheval).

3. Pansement pour une utilisation selon la revendication 1, **caractérisé en ce que** la couche de contact avec la plaie est constituée de polyuréthane, de silicone ou d'une matrice hydrocolloïdale.

4. Pansement pour une utilisation selon la revendication 1, **caractérisé en ce que** la couche de contact avec la plaie est autocollante.

5. Pansement pour une utilisation selon la revendication 1, **caractérisé en ce que** les polymères hydrophiles qui y sont intégrés sont des particules qui lient les liquides, par ex. des particules de pectine, de gélatine, de carboxyméthylcellulose ou d'un superabsorbant.

6. Pansement pour une utilisation selon la revendication 1, **caractérisé en ce que** le pansement présente un film en matière plastique respirant (semi-occlusif) en guise de couche extérieure.
